(19) Europäisches Patentamt

European Patent Office

Office européen des brevets

(11) Publication number: **0 178 895 B1**

# EUROPEAN PATENT SPECIFICATION

(12)

(45) Date of publication of patent specification: **06.03.91**   (51) Int. Cl.⁵: **A61K 33/08, A61K 9/14**

(21) Application number: **85307392.2**

(22) Date of filing: **15.10.85**

The file contains technical information submitted
after the application was filed and not included in
this specification

(54) **Fluidized and rehydratable magaldrate compositions.**

(30) Priority: **17.10.84 US 661648**
**14.08.85 US 765898**

(43) Date of publication of application:
**23.04.86 Bulletin 86/17**

(45) Publication of the grant of the patent:
**06.03.91 Bulletin 91/10**

(84) Designated Contracting States:
**AT BE CH DE FR GB IT LI LU NL SE**

(56) References cited:
**DE-A- 2 749 789**
**FR-A- 2 364 657**
**US-A- 4 117 116**

(73) Proprietor: **AMERICAN HOME PRODUCTS
CORPORATION
685, Third Avenue
New York, New York 10017(US)**

(72) Inventor: **Wu, Chien-Chin
3250, Kammerer Drive
Wilmington Delaware 19803(US)**
Inventor: **Reuter, Gerald Louis
23 Crescent Drive
Plattsburgh New York 12901(US)**
Inventor: **Coons, Mark Ernest
10 Ashline Drive
Champlain New York 12919(US)**

(74) Representative: **Wileman, David Francis et al
c/o John Wyeth and Brother Limited Hunter-
combe Lane South
Taplow Maidenhead Berkshire SL6 OPH(GB)**

EP 0 178 895 B1

EP 0 178 895 B1.

## Description

This invention relates to fluidized and rehydratable antacid compositions, more particularly to resuspendible, aqueous antacid suspension dosage forms for oral administration, to antacid powder compositions which are rehydratable to form aqueous antacid suspension dosage forms for oral administration and to methods for their preparation and use.

Antacids are widely used in the treatment of gastrointestinal disorders. Their effectiveness in promoting the healing of gastric and duodenal ulcers has been well documented. Essentially, antacids exert their positive effects by neutralizing the gastric acid secreted in the stomach. When the pH of stomach contents is raised above 3, most gastric acid is neutralized and the proteolytic activity of pepsin is inhibited. The recent elevation of antacids to a major therapeutic role, particularly in ulcer therapy, rather than a merely pallative role, has emphasized the importance of providing antacid products featuring a high neutralization (and buffer) capacity as well as a rapid rate of gastric acid neutralization. These features, particularly that of rapid rate of neutralization to the neutralization capacity of the antacid, define the more effective antacid in vivo since it is less likely that non-reacted antacids will be removed by normal gastric emptying.

Most antacids are available in both liquid and solid dosage forms. The liquid antacids, as aqueous suspensions, are, generally, more effective than the same antacids in solid dosage forms and are more commonly prescribed in the hospital. The greater effectiveness of liquid antacids is partially due to the large surface area available in liquid suspensions to react with gastric acid and partially due to the great amount of colloidal particles in aqueous suspensions which can more easily reach the affected area where treatment is needed. Moreover, aqueous suspensions of undehydrated antacids are more reactive than dry or solid antacids.

While liquid antacids possess these advantages, the same require administration of relatively large volumes of liquid suspension. The ingestion of such large volumes is inconvenient, however, making the normal problem of assuring patient compliance outside the hospital environment even more difficult. Since high dose regimens of liquid antacid have recently been shown to be effective both in promoting the healing of duodenal ulcers and in preventing the acute upper gastrointestinal bleeding in critically ill patients, the use of regular liquid antacid suspensions with the usual solid antacid content in the 6-12 percent range has become even more impractical for such therapeutic indications.

The most widely used antacids can be described as mineral type, insoluble inorganic salts that are hydrated, possess colloidal properties, and contain, for example, aluminium, magnesium and bismuth Compounds of the described mineral variety in their freshly prepared, hydrated form and in suspensions therefrom provide some of the characteristcs desired in an antacid. To provide liquid antacids with a high neutralization capacity it is necessary to increase the solids concentration of the antacid components. Such increases in concentration, however, are accompanied at higher levels with exponential increases in viscosity, a loss in colloidal properties and a loss of fluidity or mobility. Even where fluidity is initially maintained or achieved, further requirements of pharmaceutically acceptable aqueous antacid suspensions call for a smooth (non-gritty) mouth feel and maintenance of a gel structure for both suspendibility and resuspendibility. In general, resuspendible, aqueous antacid suspensions are typically de-flocculated products which contain a deflocculant or suspending agent to arrest or control further agglomeration or flocculation and settling. In the absence of a deflocculant or suspending agent type additive, the antacid in a suspension forms a hard cake or a gel structure which can no longer be resuspended with its original desirable characteristics. The formation of such a cake or gel structure is independent of antacid concentration except for low antacid concentration.

Thus deflocculants and suspending agents have been frequently included in the formulation of aqueous antacid suspensions containing solid antacid concentrations in the range of about 6 to 12 percent to prevent caking. With the growing interest in providing antacid suspension dosage forms with a greater acid neutralizing capacity, means were sought to provide highly concentrated but fluid systems.

The cost of package, shipment and storage is more expensive for liquid than solid antacids. Therefore, it would be useful to develop an antacid powder which can be reversibly converted to its original colloidal state when wetted with water.

When magaldrate is precipitated by adding the magnesium salt to the alkali aluminate solution, it is a white gel containing 6-15% solids. The spray dried powder of this gel is not rehydratable to a smooth suspension. When wetted with water, the powder remains intact and can not go back to its colloidal state. This powder appears less effective and tastes gritty. Therefore, there is a need in the art for a totally rehydratable magaldrate powder which can be easily formulated, shipped to a distant facility, stored for a period of time and reconstituted to a colloidal suspension when it is needed.

It has now been found that the liquid magaldrate compositions disclosed in this application after

2

addition of a polyhydric alcohol can be dehydrated to form a dry rehydratable magaldrate composition.

Suspensions of this invention may possess high antacid capacity and good mouth feel characteristics. Other advantages possible include rapid acid neutralization, reliably uniform reaction in acidic solutions, good fluidity, pourability and suspension characteristics and full resuspendibility under typical shelf-like conditions for a commercial aqueous antacid suspension, said advantages being retained at high concentrations.

Similar advantages can be achieved with the rehydratable compositions of this invention when admixed with water. Alternatively such rehydratable compositions may be formulated into chewable tablets with reduced grittiness when ingested.

In accordance with this invention there is provided aqueous antacid compositions characterized in providing a fluid, resuspendible, pharmaceutically elegant antacid suspension with high antacid capacity. In particular this invention provides an aqueous antacid composition in the form of a fluid, resuspendible, pharmaceutically acceptable suspension comprising precipitated and undried magaldrate gel and a fluidizing amount of a combination of an aluminium hydroxide gel having colloidal properties as a first fluidizer and potassium citrate as a second fluidizer.

In further accordance with this invention there is provided rehydratable magaldrate compositions in solid powder form which when admixed with water form aqueous antacid compositions characterized in providing a fluid, resuspendible, pharmaceutically elegant antacid suspension with high antacid capacity. In particular this invention also provides an antacid composition in solid powder form providing when admixed with water a fluid, resuspendible, pharmaceutically acceptable antacid suspension said composition comprising a co-dried combination of magaldrate gel in its freshly precipitated wet state, a polyhydric alcohol and a co-fluidizing amount of, as a first fluidizer, aluminium hydroxide gel having colloidal properties, and a co-fluidizing amount of a second fluidizer which is potassium citrate.

In another embodiment, the invention includes a method for producing aqueous antacid compositions of high antacid capacity and which are characterized in providing fluid, resuspendible pharmaceutically elegant antacid suspensions of magaldrate. Accordingly this invention provides a process for preparing an aqueous antacid composition which comprises mixing a precipitated and undried magaldrate gel, an aluminium hydroxide gel having colloidal properties as a first fluidizer and potassium citrate as a second fluidizer, the combination of fluidizers being in a fluidizing amount to form a fluid, resuspendible pharmaceutically acceptable antacid suspension. The method comprises mixing a precipitated and undried magaldrate gel with a fluidizing combination of a first and second fluidizer. The first fluidizer is selected from an aluminium hydroxide gel having colloidal properties and the second fluidizer is potassium citrate. The method for producing the high antacid capacity, aqueous antacid compositions of the invention may be broadly approached and achieved, for example, either by prior concentration of the magaldrate gel cake into a high concentration paste followed by its fluidization with a fluidizing amount of the combination of the first and second fluidizer, or, by concentration of a fluid and relatively low concentration magaldrate gel cake previously mixed with a fluidizing amount of the combination of the first and second fluidizer.

In a further embodiment, the invention includes a method for producing rehydratable antacid compositions of high antacid capacity and which are characterised in providing fluid, resuspendible pharmaceutically elegant antacid suspensions of magaldrate. Accordingly this invention also provides a process for preparing a rehydratable antacid composition which when admixed with water forms a fluid resuspendible pharmaceutically acceptable suspension said process comprising mixing a precipitated and undried magaldrate gel, an aluminium hydroxide gel having colloidal properties as a first fluidizer, a second fluidizer which is potassium citrate, and a polyhydric alcohol, and drying the resulting admixture. The method comprises mixing a precipitated and undried magaldrate gel with a fluidizing combination of a first and second fluidizer and a polyhydric alcohol. The procedure is the same as described above except that the resulting admixture is then dehydrated or dried to form the desired rehydratable antacid composition.

The magaldrate of the invention is the precipitated, undried form of the antacid. This description refers to magaldrate gel which has not been previously dried to its hydrated, anhydrous form.

Magaldrate is a chemical combination of aluminum and magnesium hydroxide, corresponding approximately to the formula $Al_5Mg_{10}(OH)_{31}(SO_4)_2xH_2O$, according to the official monograph USP XX, third supplement USP-NF, and has a molecular weight of about 1097.4. Magaldrate, also sometimes referred to in said monograph as aluminum magnesium hydroxide-sulfate, contains not less than 29.0 percent and not more than 40.0 percent of magnesium oxide (MgO) and the equivalent of not less than 18.0 percent and not more than 26.0 percent of aluminum oxide ($Al_2O_3$).

The preparation of magaldrate is described in U.S. Patent 2,923,660. A commercially suitable procedure is described in said patent, for example, beginning in column 2, line 40, although to maintain a low sodium content for the final product, the use of potassium oxide (or hydroxide) is preferred over the disclosed sodium oxide. Typically the magaldrate is precipitated to provide a 6% weight/volume mixture (fluid when fresh) and diluted to 3% for washing prior to concentration and formulation into a suspension providing a so called single strength neutralization capacity (ANC) of 13.5 to 15 meq per 5 milliliters of suspension which is equivalent to a magaldrate weight/weight concentration in the range of about 12 to 13 percent solids. At this concentration, unformulated, magaldrate is a paste-like gel. In one commercial embodiment sold under the RIOPAN trademark, the magaldrate is formulated with acacia gum to reduce the gel viscosity and achieve satisfactory fluidization. While acacia is suitable as a fluidizer for the so called single strength magaldrate product and is reported to be suitable for lowering the viscosity of a concentrated commercially aqueous antacid gel cake consisting of aluminum or magnesium hydroxide or a physical mixture of the two hydroxides, the same is not suitable for providing a magaldrate product providing greater than about 22 milliequivalents per 5 ml of ANC and the high antacid capacity composition of this invention. It is hardly surprising that the usefulness reported for acacia in liquifying aluminum or magnesium hydroxide antacids or mixtures thereof does not extend to magaldrate, since their crystal structures have been reported to be distinct. Thus, while magaldrate has a great similarity structurally to the naturally occurring minerals hydrotalcite and motukoreaite, and is believed to contain sulfate as the major interlayer, with a small amount of carbonate impurity, magnesium hydroxide is most similar to the mineral brucite and aluminum hydroxide has a disordered, amorphous polymeric structure. The physical mixtures of some aluminum and magnesium hydroxides have been reported to form hydrotalcite type structures on ageing while retaining many brucite characteristics.

Mere fluidization or deflocculation of an aqueous suspension dosage is, however, only one factor in the formulation of an aqueous antacid susension. Thus, no advantage arises from increased fluidization of more highly concentrated suspension if fluidization is achieved at the expense of resuspendibility or loss of rate or extent of acid neutralization capacity or antacid buffer capacity, immediately or upon ageing This concern is especially acute for an aqueous antacid suspension comprising magladrate as the predominant antacid because of its exceptional balance of desirable antacid properties within a single chemical entity - i.e., rapid reaction rate, prolonged buffering action within the therapeutically desired range and good acid-consuming capacity. With this invention, the desirable balance of magaldrate antacid properties is retained while the rheological properties of the magaldrate gels are significantly altered, thereby enabling the provision of concentrated, high antacid capacity magaldrate suspension. An additional benefit accompanying this invention is the uniformity of desirable rheological properties obtained which reduces or eliminates the batch to batch fine tuning frequently required to deal with the unpredictability usually inherent in the rheological properties of both fluidized and unfluidized magaldrate gels.

As previously described the magaldrate gel of this invention refers to precipitated magaldrate which has not previously been dried to its hydrated, anhydrous form. While the fluidizing combination contained in the composition of the invention may very well fluidize an aqueous mixture employing anhydrous magaldrate gel, a composition therefrom will not possess the desirable combination of suspension, resuspendibility, colloidal and antacid properties provided by the composition of the invention. Moreover, it is preferred in the composition of the invention to utilize freshly precipitated magaldrate gel since the use of older gels appears to require relatively higher proportions of the fluidizing combinations than the same composition comprising freshly precipitated magaldrate gels.

In contrast to most currently available antacid suspensions and to commercial magaldrate antacid suspensions which provide an ANC of 13.5 meq/5ml, the composition of this invention readily provides an acid neutralization capacity (double strength) of at least 25-30 meq/5ml or of at least 17-18% to 20% weight/Weight or 18 to 22% weight/volume. It will be appreciated that less concentrated suspensions such as those having an ANC of 13.5 meq/5 ml are, also, easily achievable by the composition and method of this invention. The upper limit to the ANC and concentration of the composition of this invention is only limited by the equipment available, but is believed to be on the order of 50-60 meq/5 ml.

The ratio on a dry basis of magaldrate to the fluidizing combination ranges for example from 25:1 to 2:1, and preferrably from 8:1 to 4:1. For example, in a composition having 216g magaldrate per liter of suspension, the 25:1 to 2:1 ratio would correspond to 8.0 g/l to 104 g/liter of fluidizers.

The ratio on a dry basis of the first fluidizer, aluminium hydroxide gel, calculated as aluminium oxide to the second fluidizer ranges for example from 1:15 to 1:1, preferrably from 1:6 to 1:2 and most preferrably in a range of 1:4. For example, in a composition having 216 magaldrate per liter of suspension in a ratio to fluidizers of 8:1 or 27 g/l of fluidizers the 1:4 ratio of first fluidizer to second fluidizer would correspond to 5.4 g/l of first fluidizer and 21.6 g/l of second fluidizer.

The polyhydric alcohols suitable for use in the rehydratable magaldrate compositions of this invention include those having from 2 to 6 free hydroxyl groups and include propylene glycol, glycerol,erythritol,threitol, ribitol, xylitol arabinitol, glycitol, sorbitol, mannitol and dulcitol. The polyhydric alcohol

EP 0 178 895 B1

can be present in amounts of 5 to 10% by weight of the composition. Sorbitol is the preferred polyhydric alcohol.

In one embodiment, the invention relates to a method for providing or preparing aqueous antacid compositions of high antacid capacity and which are further characterized in providing fluid, resuspendible pharmaceutically elegant antacid suspensions of magaldrate. The method broadly comprises mixing a precipitated and undried magaldrate gel with a fluidizing combination of the first and second fluidizer of this invention. Moreover, mixing can be done prior or subsequent to concentration of the magaldrate gel.

Thus in one aspect the method of this invention comprises:

(a) mixing a low concentration of a precipitated and undried magaldrate gel with a fluidizing amount of a combination of an aluminum hydroxide gel having colloidal properties as a first fluidizer and a second fluidizer which is potassium citrate;

(b) concentrating the mixture of step (a) to a fluid high antacid capacity aqueous magaldrate suspension.

Alternatively, the steps of the method of the invention may be reversed so that the magaldrate gel is first concentrated to a concentration providing a high antacid capacity and then fluidized by mixing the concentrate magaldrate gel with a fluidizing amount of a combination of the first and second fluidizer of the invention.

In a preferred embodiment the method of the invention comprises:

(a) forming an aqueous mixture containing a fluidizing amount of a combination of an aluminium hydroxide gel having colloidal properties as a first fluidizer and a second fluidizer which is potassium citrate;

(b) concentrating a precipitated and undried magaldrate gel to high antacid capacity; for example at least 10%.

(c) incrementally and continuosly charging and mixing the concentrated magaldrate gel into a stream of the mixture containing said fluidizers thereby fluidizing the concentrated gel into a fluid suspension; and

(d) incrementally and continuously charging and mixing additional concentrated magaldrate gel into a continuously recycled stream of the increasingly concentrated fluidized suspension to provide a fluidized high antacid capacity magaldrate suspension.

The concentrations and proportions of materials used in the method of the invention are the same as those described for the composition of the invention and will be based on the magaldrate concentration of the fluidized suspension. Thus, the concentration of the magaldrate in step (b) of the hereinabove described method will exceed the final concentration of the suspensions. Also, as with the composition of the invention, the method of the invention preferably employs freshly precipitated magaldrate gel.

In the rehydratable powder embodiment, the invention relates to a method for providing or preparing aqueous antacid compositions of high antacid capacity and which are further characterized in providing rehydratable magaldrate compositions which when admixed with water form fluid, resuspendible pharmaceutically elegant antacid suspensions of magaldrate. The method broadly comprises mixing a precipitated and undried magaldrate gel with a polyhydric alcohol and a fluidizing combination of the first and second fluidizer of this invention. Moreover, mixing can be done prior or subsequent to concentration of the magaldrate gel.

Thus in one aspect the method of this invention comprises:

(a) mixing a low concentration of a precipitated and undried magaldrate gel with an admixture of a polyhydric alcohol and a fluidizing amount of a combination of an aluminium hydroxide gel having colloidal properties as a first fluidizer and a second fluidizer which is potassium citrate;

(b) concentrating the mixture of step (a) to a fluid high antacid capacity aqueous magaldrate suspension.

(c) drying the mixture of step (b) as in a spray dryer.

Alternatively, the first two steps of the method of the invention may be reversed so that the magaldrate gel is first concentrated to a concentration providing a high antacid capacity and then fluidized by mixing the concentrate magaldrate gel with a fluidizing amount of a combination of the first and second fluidizer of the invention.

In a preferred embodiment the method of the invention comprises:

(a) forming an aqueous mixture containing a polyhydric alcohol and a fluidizing amount of a combination of an aluminium hydroxide gel having colloidal properties as a first fluidizer and a second fluidizer which is potassium citrate;

(b) concentrating a precipitated and undried magaldrate gel to high antacid capacity e.g. at least 10%.

(c) incrementally and continuosly charging and mixing the concentrated magaldrate gel into a stream of the mixture containing said fluidizers thereby fluidizing the concentrated gel into a fluid suspension; and

(d) drying the mixture of step (c) for example as in a spray dryer.

The composition of the invention can also and usually does include a number of pharmaceutically

5

acceptable excipients which are conventional in the aqueous antacid suspension art and do not form part of the invention. Such excipients include one or more flavoring agents e.g. peppermint; sweetening agents, e.g. saccharin, sorbitol; preservatives; sanitizers; and body building agents.

The liquid composition of this invention whether fresh or rehydrated may also contain other therapeutically active substances such as antiflatulents, as for example, simethicone; algin derivatives for treatment of esophogeal reflux; analgesics such as acetaminophen, ibuprofen and protected aspirin; various antidiarrheal or parasympatholytic agents; antiulcer agents such as cimetidine, ranitidine and sucralfate.

Compositions of this invention whether fresh or rehydrated are useful in the treatment of a variety of gastrointestinal disorders in man or animals. Typical of such disorders are hyperchlorhydria, peptic ulcer, gastric ulcer, duodenal ulcer, gastritis, esophagitis and hiatal hernia. As noted earlier, the composition of this invention are especially useful in the treatment of disorders that demand the antacid to play a therapeutic role rather than a mere palliative role. The dosage form for the composition of this invention will normally be administered orally. In general, the therapeutic dosage of the composition can be determined by relationship to the ANC of same to that of known antacid dosage forms.

While not wishing to be bound by any theory of invention, it is hypothesized that magaldrate prepared by the processes earlier described, contains small particles with a resulting greater surface area and higher surface charges. With a material such as just described, one in which it is essential to maintain the colloidal properties of the magaldrate to guarantee its most efficacious values over a useful shelf-life, control of viscosity is critical, and, is rendered more difficult in increasing concentrations. This control is achieved with a fluidizing amount of a combination of the first and second fluidizer whereby the second fluidizer apparently controls the surface charge of the magaldrate to create a desirable negative influence while the first fluidizer apparently enhances the arion absorption process and provides a competitive substrate with the magaldrate thereby maintaining an equilibrium of the negative influence. The negative influence, which can be determined by zeta potential measurements, thus fluidizes the magaldrate suspension to a mobilizable gel having an equilibrium which apparently provides for retention of desirable properties.

The invention may be further illustrated by the following examples. In Examples 1-3 the magaldrate gel (potassium base) was first concentrated to about a 24% w/w strength at which point it was a stiff immobile paste and then used to prepare the composition listed.

## Example 1

| Ingredients | Amount (mg) |
|---|---|
| Magaldrate Gel | 152.5 |
| Aluminium Hydroxide (colloidal) | 7.4 |
| Potassium Citrate | 30.8 |
| Saccharin | 0.4 |
| Sorbitol Solution (70%) | 100.0 |
| Peppermint Flavor | 0.2 |
| Orange Flavor | 2.0 |
| Water q.s. | 1000.0 |

This composition provided a magaldrate suspension according to the invention having 15.2% magaldrate solids providing approximately 20 meq/5ml of ANC.

The citrate was dissolved in sufficient water to make an 80% w/w solution. The citrate solution and the aluminium hydroxide which is provided as a paste (12.5-13.5% $Al_2O_3$) were added to the concentrated magaldrate gel paste. The mixture was blended until it became a mobile liquid suspension. The other ingredients were then added to the suspension, the water being last to provide the final weight. The mixture was further blended to yield a homogeneous suspension. The fluid suspension was then further homogenized prior to filling the palatable suspension into 12 fluid ounce container for stability observation.

The suspension was evaluated over a four month period. At each time point, the suspension was found to have maintained its initial ANC, physical appearance, particle size distribution, mobility and resuspendibility (5 hand shakes).

## Example 2

| Ingredients | Amount (mg) |
|---|---|
| Magaldrate Gel | 170.8 |
| Aluminium Hydroxide(colloidal) | 8.1 |
| Potassium Citrate | 34.2 |
| Saccharin | 0.4 |
| Sorbitol Solution (70%) | 50.0 |
| Glycerine | 50.0 |
| Peppermint flavor | 0.3 |
| Water q.s. | 1000.0 |

The magaldrate composition of the invention in this example provided a suspension having about 17.1% magaldrate solids with an approximate ANC of 24 meq/5ml.

The composition of this example was prepared as described in Example 1 and with the same results.

## Example 3

| Ingredients | Amount (mg) |
|---|---|
| Magaldrate Gel | 201.3 |
| Aluminium Hydroxide (colloidal) | 10.5 |
| Potassium Citrate | 40.1 |
| Saccharin | 0.4 |
| Sorbitol Solution (70%) | 80.0 |
| Glycerine | 20.0 |
| Lemon Flavor | 2.7 |
| Peppermint Flavor | 0.2 |
| Water q.s. | 1000.0 |

The approximate ANC of this 20.1% w/w magaldrate composition is about 29 meq/5 ml.

The composition of this example again provided a fluid suspension and was prepared as described in Example 1 with the same results.

Example 4

A magaldrate suspension product, according to the invention, having an ANC of 30 meq/5ml or magaldrate 1080 mg/5ml of suspension was prepared as follows. The formula given is for 1 liter (1.18Kg).

7

| Ingredient | Amount |
|---|---|
| Magaldrate Gel | 2.93 l* |
| Aluminium Hydroxide Gel (12.5% Al$_2$O$_3$) | 47.8 g |
| Potassium Citrate | 19.6 g |
| Sorbitol Solution (70%) | 57.4 g |
| Glycerin | 47.8 g |
| Saccharim | 0.383 g |
| Xanthan Gum | 1.43 g |
| Peppermint, Natural and Artificial | 0.283 ml |
| Monochloramine Solution | q.s. |
| Water, purified, Chlorinated q.s. | 1.18 Kg. |

**\* Theoretical input per liter is 216g Magaldrate at 100%**

1. In a suitable tank equipped with a mixer, the sorbitol solution, 15.7g of the water and the citrate were combined and then mixed until a clear solution was obtained. The aluminium hydroxide gel was added and mixed until uniform and mixing continued until use.

2. Immediately before concentration of the magaldrate gel, 126g of the Step #1 mixture was added to a jacketed tank equipped with a stirrer. With continuous stirring, the magaldrate gel was concentrated on a rotary filter to a concentration of not less than 24% w/w into the mixture and cooling to 25° C was begun. After all the concentrated had been added the remainder of the Step #1 mixture was added with continuous stirring to achieve uniformity.

3. The Step #2 mixture was poured through a homogenizer into a jacketed tank with continuous stirring and continuous cooling to 25° C.

4. The remaining ingredients, except the water and monochloramine, were mixed in a separate container until uniform, after which, they were added to the magaldrate mixture and mixed until the xanthan gum was hydrated. Water was then added (with mixing) to bring the batch up to about 1.18 Kg.

5. Prior to filling the suspension, sufficient monochloramine was added to and mixed into the bulk material to provide at least 85 ppm in the suspension.

6. The suspension was then filled into sterilized containers which were capped, inverted and returned to the upright position.

The suspension of this example was subjected to repeated freeze-thaw cycles of 24 hours each, and after each cycle both the physical and antacid properties were retained. In contrast, a conventional magaldrate suspension at even 13.5 meq/5ml with acacia as the deflocculant did not retain all the properties after only one such cycle.

Example 5

Using the procedure described in Example 4, a magaldrate suspension, according to the invention, having an ANC of about 45 meq/5ml may be prepared.

EP 0 178 895 B1

| Ingredients | Amount |
|---|---|
| Magaldrate Gel (at about 34%) | 985.0 g |
| Aluminium Hydroxide Gel (Al$_2$O$_3$12.5%) | 47.8 g |
| Potassium Citrate | 39.2 g |
| Sorbitol Solution (70%) | 57.4 g |
| Glycerin | 47.8 g |
| Saccharin | 0.383 g |
| Xanthan Gum | 1.43 g |
| Peppermint Flavor | 0.283 ml |
| Monochloramime Solution q.s. | 100 ppm |
| Water, purified, chlorinated q.s. ad | 1.0 L |

Example 6

A magaldrate suspension, according to the invention, was prepared with the formula and procedure described in Example 4, but for the addition of simethicone to provide a suspension with an ANC of 30 meq/5ml and 30 mg/5ml of simethicone.

## Example 7

| Ingredient | A | B | Amount | |
|---|---|---|---|---|
| | | | A | B |
| Magaldrate Gel at 27.6% w/w or 24.14% w/w/ | | | 1565g | 1789g |
| Aluminum Hydroxide Gel (13.5% Al$_2$O$_3$) | | | 86.04 | 100.4g |
| Potassium Citrate Solution (80%) | | | 61.9 | 137.6 g |
| Sorbitol Solution (70%) | | | 114.8 g | |
| Glycerin | | | 95.6 g | |
| Saccharin | | | .766g | |
| Xanthan Gum | | | 2.86 | 3.43 g |
| Peppermint Flavor | | | 0.57 ml | |
| Monochloramine Solution q.s | | | 100 ppm | |
| Water, distilled q.s. | | | 2.321 Kg | |

In this experiment, two different freshly concentrated magaldrate gels at the percents indicated were employed to prepare suspensions with an ANC of 30 meq/5ml. The sorbitol, citrate, and aluminum hydroxide gel were added to a container equipped with a stirrer and mixing was begun. The magaldrate was added to the same container, preferrably incrementally and mixed until uniform. The saccharin and flavor were dispensed in the glycerin; after which, the xanthan gum was added and mixed until uniform. The flavored dispension was then added to the fluidized magaldrate with mixing until the mixture was uniform and the gum hydrated. Sufficient distilled water was added with mixing as was a quantity of the chlorine sanitizing solution to provide about 100 ppm chlorine. The suspensions (12) having an aluminum oxide ratio tc citrate ranging from about 1:8.8 to about 1:3.95 were then filled into container. All formulations provided

9

suspensions with the desired physical and antacid properties including resuspendibility.

Example 8

A typical formulation for the monochloramine solution referred to in some

| Calcium Hypochlorite 60% | 14.0 g |
| Ammonia Solution, Strong | 7.4 g |
| Water, purified, chlorinated | 1998. g |

of the prior examples is as follows:

In the following examples a Buchi 190 Mini Spray Dryer was employed to dry the initial admixture containing magaldrate, the polyhydric alcohol and the first and second fluidizers. This model spray dryer is manufactured by Buchi Laboratoriums-Technik AG. Other spray dryers can be employed, however, such as those manufactured by Anhydro Company of Attleboro, Massachusetts and Niro Atomizer Inc., of Columbia, Maryland, so long as the spray dryer can process the relatively viscous admixture. The operating conditions for the spray dryer are customarily an inlet temperature of 130°C, an outlet temperature of 400°C and a wheel speed of 20,000 RPM.

EXAMPLE 9

In this example, a rehydratable magaldrate powder of this invention was prepared having the following formulation:

| INGREDIENTS | AMOUNT |
|---|---|
| Magaldrate Gel Sulfate, Potassium Based ( 7.38%) | 90.0 kg |
| Potassium Citrate, NF | 1.47 kg |
| Aluminium Hydroxide Gel, Guilini, A671/4 | 1.85 kg |
| Sorbitol Solution, USP | 2.85 kg |

The above listed ingredients were processed as set forth below. Continuous agitation must be maintained throughout the processing.

Step 1. Add the sorbitol solution, USP to a suitable tank equipped with a stirrer.

Step 2. Add the potassium citrate, NF then mix until uniform.

Step 3. Concentrate the magaldrate gel sulfate, potassium base to not less than 24% magaldrate and add to the tank in Step #2

Step 4. When approximately half of the concentrated gel has been added to the batch, add the aluminum hydroxide gel to the mixture of Step #3.

Step 5. When all of the gel has been added, obtain an assay of the magaldrate content and adjust the quantities of all ingredients to the theoretical ratios. Mix for 5 minutes after each addition.

Step 6. Spray dry the Step #5 gel mixture at the following conditions:

| Inlet | 400°C |
| Outlet | 130°C |
| Wheel Speed | 20,000 RPM |

## EXAMPLE 10

In this example, the rehydratable magaldrate powder of Example 9 was reconstituted into a stable, liquid magaldrate formulation for oral administration containing 15 milliequivalents of magaldrate per 5 milliliters of formulation. In this and the following examples the monochloroamine solution employed was a stock solution prepared in the following manner from the listed ingredients.

### Monochloramine Solution

| INGREDIENTS | AMOUNT |
| --- | --- |
| Calcium Hypochlorite, 60% | 14.00 g |
| Ammonia Solution, Strong, NF | 7.4 g |
| Water, Purified, USP, Chlorinated | 28.0 g |
| Water, Purified, USP, Chlorinated | 1.97 kg |

The calcium hypochlorite, 60% was added to 1.97 kg water and mixed with vigorous stirring for at least 10 minutes. The ammonia solution was added to 28.0 g water and stirred briefly. The ammonia solution was then added to the chlorine solution and mixed with vigorous stirring until uniform and filtered after an hour.

Using the rehydratable magaldrate powder as prepared in Example 9, the monochloroamine solution prepared as shown above, and the other ingredients shown below, the reconstituted magaldrate was prepared as follows:

| INGREDIENTS | AMOUNT |
| --- | --- |
| Rehydratable Magaldrate, Spray Dried 64% w/w | 168.75 g* |
| Glycerin, USP | 23.9 g |
| Saccharin, NF | 0.383 g |
| Peppermint Flavor #5917 | 0.283 ml |
| Xanthan Gum, FCC | 3.0 g |
| Monochloramine Soln. QS | QS 100 PPM |
| Water Purified, USP Chlorinated | QS 1000 ml = 1100 g |

*Theoretical input per liter is 108 g Magaldrate 100%

THe above listed ingredients were processed as set forth below. During processing the equipment should be kept clean to minimize bacterial growth and the processing carried out as aseptically as practicable. The finished suspension should be kept in a closed tank to minimize monochloramine losses during filling. Also continuous stirring must be maintained throughout.

Step 1. Place 700 grams of chlorinated water into a suitable compounding tank equipped with a mixer.

Step 2. Slowly add the magaldrate powder with constant mixing, mix for approximately 1 hours or until completely hydrated.

Step 3. To a separate container equipped with an agitator, add the Glycerin, saccharin, xanthan gum, and the peppermint flavor and mix until uniform, then cool to 30° C or less.

Step 4. Add the Step #3 mixture to the Step #2 mixture and mix until the xanthan gum is hydrated.

Step 5. Add sufficient water to bring the batch to 1.0774 kg.

Step 6. Pass the suspension through a homogenizer at 1000 PSIG ± l00 into a jacketed tank with continuous stirring.

Step 7. Immediately prior to the start of filling, add monochloramine at 6,000 PPM to achieve 100 PPM (NLT 85) by assay in the bulk suspension and mix for 30 minutes. The suspension of step 7 should be below 25°C before adding the monochloroamine.

Step 8. Fill the finished suspension into bottles with screw cap which may have been previously sterilized.

Step 9. Invert each bottle of suspension for not less than 2 seconds (NMT 1 hour), then return to the upright position.

EXAMPLE 11

In this example, the rehydratable magaldrate powder of Example 9 was reconstituted into a stable, liquid magaldrate formulation for oral administration containing 30 milliequivalents of magaldrate per 5 milliliters of formulation.

Using the rehydratable magaldrate powder as prepared in Example 9 the monochloroamine solution prepared as shown in Example 10,and the other ingredients shown below, the reconstituted magaldrate was prepared as follows:

| INGREDIENTS | AMOUNT |
|---|---|
| Rehydratable Magaldrate, Spray Dried 64% w/w | 337.55 g* |
| Glycerin, USP | 47.8 g |
| Simethicone, USP 95% w/w | 6.32 g |
| Saccharin, NF | 0.383 g |
| Peppermint Flavor #5917 | 0.283 ml |
| Xanthan Gum, FCC | 3.5 g |
| Monochloramine Soln. QS | QS 100 PPM |
| Water Purified, USP Chlorinated | QS 1000 ml = 1200 g 100% |

*Theoretical input per liter is 216g Magaldrate

The above listed ingredients were processed in the same manner as in Example 10 except that in Step 1 only 500 grams of chlorinated water was placed in the mixer, the simethicone was added and mixed after Step 4, and in Step 5, sufficient water was added to bring the batch only to 1.0774 kg.

EXAMPLE 12

In this example, a rehydratable magaldrate composition of this invention was prepared having the following formulation to produce 100 kg of magaldrate powder.

| INGREDIENTS | AMOUNT |
|---|---|
| Magaldrate Gel Sulfate, Potassium Based ( 7.38%) | 908 kg |
| Potassium Citrate, NF, Granular | 9.4 kg |
| Aluminum Hydroxide Gel, Giulini A671/4 12% (Obtained from Giulini Chemie,GmbH, Ludwigschaffen 15,Guilinistrasse 3, P.O.Box 123,W.Germany ) | 11.8 kg |
| Sorbitol Solution, USP 70% | 29.4 kg |
| Water, Purified, USP, Chlorinated | 5.7 kg |

Typical condition for spray drying:

Inlet Temperature – 400°C

Outlet Temperature – 130°C

Atomization Wheel Speed 16,000 RPM

Magaldrate concentration of resultant powder would typically range from 65-68%.

EXAMPLE 13

A magaldrate chew tablet prepared from the rehydratable powder of Example 12 sufficient to make 1000 directly compressed tablets is shown below:

| INGREDIENTS | AMOUNT |
|---|---|
| Magaldrate Rehydratable Spray Dried Powder    67% | 806.0 g |
| Mannitol, USP | 290.5 g |
| Nu-tab (a directly compressible sugar) | 290.5 g |
| Flavor-Spearmint, Aromalok | 6.0 g |
| Magnesium Stearate | 7.0 g |
| Theoretical Tablet Weight | 1,400 mg |

Other examples of directly compressible tabletting ingredients for this product would be dextrates, sorbitol and other directly compressible sugars such a Di-Pac, a compressible sucrose made by Amstar Corporation of New York, N.Y. Zinc Stearate could also be substituted as a lubricant for magnesium stearate. A lozenge dosage form rather than a chew tablet can be prepared using suitable tabletting ingredients such as sorbitol.

EXAMPLE 14 Magaldrate rehydratable granules can be prepared from the rehydratable powder of Example 12 to provide 1 kilogram of granulation as follows

| INGREDIENTS | AMOUNT |
|---|---|
| Magaldrate Rehydratable Spray | |
| Dried Powder 67% | 700.0 g |
| Sugar Confectioners, NF | 300.0 g |
| Water, Purified, USP, Chlorinated | 150.0 ml |

The above powders are wet massed in a suitable mixer by the addition of water. The resultant granulation is wet sized, dried in a fluidized bed dryer and then dry sized. The potency of the above resultant granulation is 46.9% magaldrate.

EXAMPLE 15

The rehydratable magaldrate granules as prepared in Example 14 can be packaged to provide a 30 milliequivalent magaldrate dose per 5 milliliters of oral dosage form as follows:

| INGREDIENTS | AMOUNT |
|---|---|
| Magaldrate Rehydratable Granules | |
| 46.9% | 2.3 g |
| Flavor, Spearmint, Aromalok | 0.004 g |

To the above blend, one would add 5 ml water and shake to rehydrate. This would give a single 30 mEq dose.

EXAMPLE 16

The rehydratable magaldrate granules as prepared in Example 14 can also be packaged with milk solids to provide a 30 milliequivalent magaldrate dose per 20 milliliters of oral dosage form as follows:

| INGREDIENTS | AMOUNT |
|---|---|
| Magaldrate Rehydratable Granules | |
| 46.9% | 2.3 g |
| Milk Solids (Instant Non-Fat Dry Milk) | 0.7 g |
| Flavor-Spearmint, Aromalok | 0.006 g |

Again for a single dose, 20 ml of water would be added to the above blend to allow for rehydration. The milk solids are added to give a creamier and more pleasant tasting product.

EXAMPLE 17

The rehydratable magaldrate granules as prepared in Example 14 can further be packaged with other pharmaceutical agents such as antidiarrheal agents, i.e. adsorbents and anticholinergics, as follows:

14

| INGREDIENTS | AMOUNT |
|---|---|
| Magaldrate Rehydratable Granules 46.9% | 1,150 mg |
| Kaolin (hydrated aluminum silicate) Adsorbent | 1,000 mg |
| Hyoscyamine Sulfate ⎱ anticholinergics | 0.035 mg |
| Hyoscine Hydrobromide ⎰ | 0.002 mg |

The mixture would be rehydrated and resuspended with 20 ml water for a single dose (ANC of 15 mEq).

The packaging of the compositions of Examples 15-17 preferably would be of the foil or hermetic unit type.

Additional formulation of rehydratable magaldrate powder are shown in Table 1.

15

T A B L E   1

In each of the formulae, one kilogram of 13.6% magaldrate gel fresh off the artisan was employed. The other ingredients are as shown in grams.

| Formula | 1 | 2 | 3 | 4 | 5 | 6 | 7 | 8 | 9 | 10 | 11 | 12 |
|---|---|---|---|---|---|---|---|---|---|---|---|---|
| Potassium Citrate | 19.12 | 15 | 10 | 25 | 30 | 5 | 19.72 | 30 | 19.72 | 19.12 | 10 | 30 |
| $Al(OH)_3$ Fluid Gel | 24.89 | 18.9 | 12.62 | 31.55 | 37.86 | 37.86 | 24.89 | 37.86 | 24.89 | 24.89 | 12.62 | 37.86 |
| Sorbitol 100% | 58.29 | 58.29 | 58.29 | 58.29 | 58.29 | 58.29 | 77.71 | 77.71 | 97.14 | 58.29 | 58.29 | 58.29 |
| Saccharin NF | --- | --- | --- | --- | --- | --- | --- | --- | --- | .383 | .383 | .383 |

PROCEDURE:

1. To the small Hobart mixing bowl add the magaldrate gel, potassium citrate, $Al(OH)_3$, sorbitol, and saccharin NF, if in formula and mix until uniform.

2. Add 100 PPM monochlormine to Step No. 1 and mix.

3. Dry on Buchi 190 Mini Spray Dryer.

Of the rehydratable magaldrate powder compositions of Table 1, Formula 8 gave the best mouth feel in terms of no grittiness and Formula 6 was next best. All of the formulas were readily rehydratable.

16

**Claims**

1. An aqueous antacid composition in the form of a fluid, resuspendible, pharmaceutically acceptable suspension comprising precipitated and undried magaldrate gel and a fluidizing amount of a combination of an aluminium hydroxide gel having colloidal properties as a first fluidizer and potassium citrate as a second fluidizer

2. An antacid composition in solid powder form providing when admixed with water a fluid, resuspendible, pharmaceutically acceptable antacid suspension said composition comprising a co-dried combination of magaldrate gel in its freshly precipitated wet state, a polyhydric alcohol and a co-fluidizing amount of, as a first fluidizer, aluminium hydroxide gel having colloidal properties, and a co-fluidizing amount of a second fluidizer which is potassium citrate.

3. A composition as claimed in Claims 1 or 2 wherein the ratio on a dry basis of magaldrate to the combination of fluidizers ranges from 25:1 to 2:1

4. A composition as claimed in any one of Claims 1 to 3 wherein the ratio on a dry basis of the first fluidizer to the second fluidizer ranges from 1:15 to 1:1.

5. A composition as claimed in Claim 1 or Claim 2 wherein the ratio on a dry basis of magaldrate to the combination of fluidizers is 8:1 to 4:1 and the ratio on a dry basis of the first fluidizer to the second fluidizer is 1:6 to 1:2.

6. A composition as claimed in any one of Claims 1 to 5 comprising at least 10% w/v of said magaldrate when in suspension.

7. A composition as claimed in any one of Claims 1 to 5 comprising at least 18% w/v of said magaldrate when in suspension.

8. A composition as claimed in any one of Claims 2 to 7 in which the polyhydric alcohol contains 2 to 6 free hydroxy groups.

9. A composition as claimed in any one of Claims 2 to 7 wherein the polyhydric alcohol is sorbitol.

10. A process for preparing an aqueous antacid composition which comprises mixing a precipitated and undried magaldrate gel an aluminium hydroxide gel having colloidal properties as a first fluidizer and potassium citrate as a second fluidizer, the combination of fluidizers being in a fluidizing amount to form a fluid, resuspendible pharmaceutically acceptable antacid suspension.

11. A method as claimed in Claim 10 in which prior to mixing with the fluidizers, said magaldrate gel is in the form of a stiff and non-flowing paste.

12. A method as claimed in Claim 10 in which prior to mixing with the fluidizers, said magaldrate gel is in a dilute concentration and after mixing with the fluidizers said aqueous mixture is concentrated by removing water.

13. A method according to Claim 10 comprising the steps of:
    (a) forming an aqueous mixture containing a fluidizing amount of a combination of an aluminium hydroxide gel having colloidal properties as a first fluidizer and a potassium citrate as a second fluidizer.
    (b) concentrating a precipitated and undried magaldrate gel to a weight/volume concentration of at least 10%;
    (c) incrementally and continuously charging and mixing the concentrated magaldrate gel into a stream of the mixture containing said fluidizer thereby fluidizing the concentrated gel into a fluid suspension; and
    (d) incrementally and continuously charging and mixing additional concentrated magaldrate gel into a continuously recycled stream of the increasingly concentrated fluidized suspension to provide a fluidized high antacid capacity magaldrate suspension.

17

14. A process for preparing a rehydratable antacid composition which when admixed with water forms a fluid resuspendible pharmaceutically acceptable suspension said process comprising mixing a precipitated and undried magaldrate gel, an aluminium hydroxide gel having colloidal properties as a first fluidizer, potassium citrate as a second fluidizer and a polyhydric alcohol, and drying the resulting admixture; the combination of fluidizers being a fluidizing amount.

15. A process according to Claim 14 comprising the steps of:
(a) forming an aqueous mixture containing a polyhydric alcohol and a fluidizing amount of a combination of an aluminium hydroxide gel having colloidal properties as a first fluidizer and potassium citrate as a second fluidizer;
(b) concentrating a precipitated and undried magaldrate gel to a weight/volume concentration of at least 10%;
(c) incrementally and continuously charging and mixing the concentrated magaldrate gel into a stream of the mixture containing said fluidizer thereby fluidizing the concentrated gel into a fluid suspension; and
(d) drying the admixture of magaldrate gel and first and second fluidizer.

CLAIMS for the Contracting State AT

1. A process for preparing an aqueous antacid composition which comprises mixing a precipitated and undried magaldrate gel, an aluminium hydroxide gel having colloidal properties as a first fluidizer and potassium citrate as a second fluidizer, the combination of fluidizers being in a fluidizing amount to form a fluid, resuspendible pharmaceutically acceptable antacid suspension.

2. A process as claimed in Claim 1 in which prior to mixing with the fluidizers, said magaldrate gel is in the form of a stiff and non-flowing paste.

3. A process as claimed in Claim 1 in which prior to mixing with the fluidizers, said magaldrate gel is in a dilute concentration and after mixing with the fluidizers said aqueous mixture is concentrated by removing water.

4. A process for according to Claim 1 comprising the steps of:
(a) forming an aqueous mixture containing a fluidizing amount of a combination of an aluminium hydroxide gel having colloidal properties as a first fluidizer and potassium citrate as a second fluidizer;
(b) concentrating a precipitated and undried magaldrate gel to a weight/volume concentration of at least 10%;
(c) incrementally and continuously charging and mixing the concentrated magaldrate gel into a stream of the mixture containing said fluidizer thereby fluidizing the concentrated gel into a fluid suspension; and
(d) incrementally and continuously charging and mixing additional concentrated magaldrate gel into a continuously recycled stream of the increasingly concentrated fluidized suspension to provide a fluidized high antacid capacity magaldrate suspension.

5. A process for preparing a rehydratable antacid composition which when admixed with water forms a fluid resuspendible pharmaceutically acceptable suspension said process comprising mixing a precipitated and undried magaldrate gel, an aluminium hydroxide gel having colloidal properties as a first fluidizer, potassium citrate as a second fluidizer, and a polyhydric alcohol, and drying the resulting admixture; the combination of fluidizers being a fluidizing amount.

6. A process according to Claim 5 comprising the steps of:
(a) forming an aqueous mixture containing a polyhydric alcohol and a fluidizing amount of a combination of an aluminium hydroxide gel having colloidal properties as a first fluidizer and potassium citrate as a second fluidizer;
(b) concentrating a precipitated and undried magaldrate gel to a weight/volume concentration of at least 10%;
(c) incrementally and continuously charging and mixing the concentrated magaldrate gel into a stream of the mixture containing said fluidizer thereby fluidizing the concentrated gel into a fluid suspension; and

(d) drying the admixture of magaldrate gel and first and second fluidizer.

7. A process as claimed in any one of Claims 1 to 6 wherein the ratio on a dry basis of magaldrate to the combination of fluidizers ranges from 25:1 to 2:1.

8. A process as claimed in any one of Claims 1 to 6 wherein the ratio on a dry basis of the first fluidizer to the second fluidizer ranges from 1:15 to 1 :1

9. A process as claimed in any one of Claims 1 to 6 wherein the ratio on a dry basis of magaldrate to the combination of fluidizers is about 8:1 to about 4:1 and the ratio on a dry basis of the first fluidizer to the second fluidizer is 1 :6 to 1 :2.

10. A process as claimed in any one of Claims 5 to 9 in which the polyhydric alcohol contains 2 to 6 free hydroxy groups.

11. A process as claimed in any one of Claims 5 to 9 wherein the polyhydric alcohol is sorbitol.

12. A process as claimed in any one of Claims 1 to 11 in which the magaldrate comprises at least 10% w/v when in suspension.

13. A process as claimed in any one of Claims 1 to 11 in which the magaldrate comprises at least 18% w/v when in suspension.

## Revendications

1. Composition anti-acide aqueuse sous forme d'une suspension fluide, pouvant être remise en suspension, pharmaceutiquement acceptable, comprenant un gel de magaldrate précipité et non déshydraté et une quantité fluidifiante d'un mélange d'un gel d'hydroxyde d'aluminium ayant des propriétés colloïdales, servant de premier fluidifiant, et de citrate de potassium servant de second fluidifiant.

2. Composition anti-acide sous forme de poudre solide donnant, lors de son mélange à de l'eau, une suspension anti-acide fluide, pouvant être remise en suspension, pharmaceutiquement acceptable, ladite composition comprenant un mélange, ayant subi une co-déshydratation, d'un gel de magaldrate a l'état humide fraîchement précipité, d'un polyalcool, d'une quantité co-fluidifiante d'un gel d'hydroxyde d'aluminium ayant des propriétés colloïdales, servant de premier fluidifiant, et d'une quantité co-fluidifiante d'un second fluidifiant qui est le citrate de potassium.

3. Composition suivant la revendication 1 ou 2, dans laquelle le rapport, sur base sèche, du magaldrate au mélange de fluidifiants va de 25:1 à 2:1.

4. Composition suivant l'une quelconque des revendications 1 à 3, dans laquelle le rapport, sur base sèche, du premier fluidifiant au second fluidifiant va de 1:15 à 1:1.

5. Composition suivant la revendication 1 ou la revendication 2, dans laquelle le rapport, sur base sèche, du magaldrate au mélange de fluidifiants va de 8:1 à 4:1 et le rapport, sur base sèche, du premier fluidifiant au second fluidifiant va de 1:6 à 1:2.

6. Composition suivant l'une quelconque des revendications 1 à 5, comprenant au moins 10 % en poids/vo lume de magaldrate, lorsque ce dernier est en suspension.

7. Composition suivant l'une quelconque des revendications 1 à 5, comprenant au moins 18 % en poids/volume de magaldrate, lorsque ce dernier est en suspension.

8. Composition suivant l'une quelconque des revendications 2 à 7, dans laquelle le polyalcool contient 2 à 6 groupes hydroxy libres.

EP 0 178 895 B1

**9.** Composition suivant l'une quelconque des revendications 2 à 7, dans laquelle le polyalcool est le sorbitol.

**10.** Procédé de préparation d'une composition anti-acide aqueuse, qui consiste à mélanger un gel de magaldrate précipité et non déshydraté, un gel d'hydroxyde d'aluminium ayant des propriétés colloïdales, servant de premier fluidifiant, et de citrate de potassium servant de second fluidifiant, le mélange de fluidifiants étant présent en une quantité fluidifiante pour former une suspension anti-acide fluide, pouvant être remise en suspension, pharmaceutiquement acceptable.

**11.** Procédé suivant la revendication 10, dans lequel, avant le mélange aux fluidifiants, le gel de magaldrate est sous forme d'une pâte rigide et non apte à l'écoulement.

**12.** Procédé suivant la revendication 10, dans lequel, avant le mélange aux fluidifiants, le gel de magaldrate est à l'état dilué et, après mélange aux fluidifiants, le mélange aqueux est concentré par élimination de l'eau.

**13.** Procédé suivant la revendication 10, comprenant les étapes consistant :
(a) à former un mélange aqueux contenant une quantité fluidifiante d'une association d'un gel d'hydroxyde d'aluminium ayant des propriétés colloïdales, servant de premier fluidifiant, et de citrate de potassium servant de second fluidifiant ;
(b) à concentrer un gel de magaldrate précipité et non déshydraté à une concentration en poids/volume d'au moins 10 % ;
(c) à introduire et à mélanger, par portions successives et en continu, le gel de magaldrate concentré dans un courant du mélange contenant ledit fluidifiant, ce qui permet de transformer par fluidification le gel concentré en une suspension fluide ; et
(d) à introduire et à mélanger, par portions successives et en continu, une quantité supplémentaire de gel de magaldrate concentré dans un courant recyclé en continu de la suspension fluidifiée à concentration croissante pour produire une suspension fluidifiée de magaldrate à fort pouvoir anti-acide.

**14.** Procédé de préparation d'une composition anti-acide réhydratable qui, lors de son mélange à de l'eau, forme une suspension fluide, pouvant être remise en suspension, pharmaceutiquement acceptable, ledit procédé consistant à mélanger un gel de magaldrate précipité et non déshydraté, un gel d'hydroxyde d'aluminium ayant des propriétés colloïdales, servant de premier fluidifiant, du citrate de potassium servant de second fluidifiant et un polyalcool, et à déshydrater le mélange résultant ; l'association de fluidifiants étant présente en une quantité fluidifiante.

**15.** Procédé suivant la revendication 14, comprenant les étapes consistant :
(a) à former un mélange aqueux contenant un polyalcool et une quantité fluidifiante d'une association d'un gel d'hydroxyde d'aluminium ayant des propriétés colloïdales, servant de premier fluidifiant, et de citrate de potassium servant de second fluidifiant ;
(b) à concentrer un gel de magaldrate précipité et non déshydraté à une concentration en poids/volume d'au moins 10 % ;
(c) à introduire et à mélanger, par portions successives et en continu, le gel de magaldrate concentré dans un courant du mélange contenant ledit fluidifiant, ce qui permet de transformer par fluidification le gel concentré en une suspension fluide ; et
(d) à déshydrater le mélange de gel de magaldrate et des premier et second fluidifiants.

REVENDICATIONS pour 1'Etat contractant : AT

**1.** Procédé de préparation d'une composition anti-acide aqueuse, qui consiste à mélanger un gel de magaldrate précipité et non déshydraté, un gel d'hydroxyde d'aluminium ayant des propriétés colloïdales, servant de premier fluidifiant, et du citrate de potassium servant de second fluidifiant, l'association de fluidifiants étant présente en une quantité fluidifiante pour former une suspension anti-acide fluide, pouvant être remise en suspension, pharmaceutiquement acceptable.

**2.** Procédé suivant la revendication 1, dans lequel, avant le mélange aux fluidifiants, le gel de magaldrate est sous forme d'une pâte rigide et non apte à l'écoulement.

20

3. Procédé suivant la revendication 1, dans lequel, avant le mélange aux fluidifiants, le gel de magaldrate est présent à l'état dilué et, après mélange aux fluidifiants, le mélange aqueux est concentré par élimination de l'eau.

4. Procédé suivant la revendication 1, comprenant les étapes consistant :

(a) à former un mélange aqueux contenant une quantité fluidifiante d'une association d'un gel d'hydroxyde d'aluminium ayant des propriétés colloïdales, servant de premier fluidifiant, et de citrate de potassium servant de second fluidifiant ;

(b) à concentrer un gel de magaldrate précipité et non déshydraté à une concentration en poids/volume d'au moins 10 % ;

(c) à introduire et à mélanger, par portions successives et en continu, le gel de magaldrate concentré dans un courant du mélange contenant ledit fluidifiant, ce qui permet de transformer par fluidification le gel concentré en une suspension fluide ; et

(d) à introduire et à mélanger, par portions successives et en continu, une quantité supplémentaire de gel de magaldrate concentré dans un courant recyclé en continu de la suspension fluidifiée à concentration croissante pour produire une suspension fluidifiée de magaldrate à fort pouvoir anti-acide.

5. Procédé de préparation d'une composition anti-acide réhydratable qui, lors de son mélange à de l'eau, forme une suspension fluide, pouvant être remise en suspension, pharmaceutiquement acceptable, ledit procédé consistant à mélanger un gel de magaldrate précipité et non déshydraté, un gel d'hydroxyde d'aluminium ayant des propriétés colloïdales, servant de premier fluidifiant, du citrate de potassium servant de second fluidifiant et un polyalcool, et à déshydrater le mélange résultant ; l'association de fluidifiants étant présente en une quantité fluidifiante.

6. Procédé suivant la revendication 5, comprenant les étapes consistant :

(a) à former un mélange aqueux contenant un polyalcool et une quantité fluidifiante d'une association d'un gel d'hydroxyde d'aluminium ayant des propriétés colloïdales, servant de premier fluidifiant, et de citrate de potassium servant de second fluidifiant ;

(b) à concentrer un gel de magaldrate précipité et non déshydraté à une concentration en poids/volume d'au moins 10 % ;

(c) à introduire et à mélanger, par portions successives et en continu, le gel de magaldrate concentré dans un courant du mélange contenant ledit fluidifiant, ce qui permet de transformer par fluidification le gel concentré en une suspension fluide ; et

(d) à déshydrater le mélange de gel de magaldrate et des premier et second fluidifiants.

7. Procédé suivant l'une quelconque des revendications 1 à 6, dans lequel le rapport, sur base sèche, du magaldrate à l'association de fluidifiants va de 25:1 à 2:1.

8. Procédé suivant l'une quelconque des revendications 1 à 6, dans lequel le rapport, sur base sèche, du premier fluidifiant au second fluidifiant va de 1:15 à 1:1.

9. Procédé suivant l'une quelconque des revendications 1 à 6, dans lequel le rapport, sur base sèche, du magaldrate à l'association de fluidifiants va d'environ 8:1 à environ 4:1 et le rapport, sur base sèche, du premier fluidifiant au second fluidifiant va de 1:6 à 1:2.

10. Procédé suivant l'une quelconque des revendications 5 à 9, dans lequel le polyalcool contient 2 à 6 groupes hydroxy libres.

11. Procédé suivant l'une quelconque des revendications 5 à 9, dans lequel le polyalcool est le sorbitol.

12. Procédé suivant l'une quelconque des revendications 1 à 11, dans lequel le magaldrate est présent en une quantité d'au moins 10 % en poids/volume, lorsqu'il est en suspension.

13. Procédé suivant l'une quelconque des revendications 1 à 11, dans lequel le magaldrate est présent en une quantité d'au moins 18 % en poids/volume, lorsqu'il est en suspension.

**Ansprüche**

1. Wässerige Säureneutralisatorzusammensetzung in Form einer fluiden, resuspendierbaren pharmazeutisch annehmbaren Suspension, die ausgefälltenes und ungetrocknetes Magaldratgel und eine fluidisierende Menge einer Kombination eines Aluminiumhydroxidgels mit kolloidalen Eigenschaften als erstes Fluidisierungsmittel und Kaliumcitrat als zweites Fluidisierungsmittel umfaßt.

2. Säureneutralisatorzusammensetzung in fester Pulverform die, wenn sie mit Wasser gemischt wird, eine fluide, resuspendierbare pharmazeutisch annehmbare Säureneutralisatorsuspension ergibt, welche Zusammensetzung eine gemeinsam getrocknete Kombination von Magaldratgel in seinem frisch ausgefällten feuchten Zustand, eines mehrwertigen Alkohols und einer cofluidisierenden Menge von Aluminiumhydroxidgel mit kolloidalen Eigenschaften als erstes Fluidisierungsmittel und einer cofluidisierenden Menge eines zweiten Fluidisierungsmittels, das Kaliumcitrat ist, umfaßt.

3. Zusammensetzung nach Anspruch 1 oder 2, worin auf Trockenbasis das Verhältnis von Magaldrat zu der Kombination der Fluidisierungsmittel 25 : 1 bis 2 : 1 beträgt.

4. Zusammensetzung nach einem der Ansprüche 1 bis 3, worin auf Trockenbasis das Verhältnis des ersten Fluidisierungsmittels zum zweiten Fluidisierungsmittel 1 : 15 bis 1 : 1 beträgt.

5. Zusammensetzung nach Anspruch 1 oder 2, worin auf Trockenbasis das Verhältnis von Magaldrat zu der Kombination der Fluidisierungsmittel 8 : 1 bis 4 : 1 und auf Trockenbasis das Verhältnis des ersten Fluidisierungsmittels zum zweiten Fluidisierungsmittel 1 : 6 bis 1 : 2 beträgt.

6. Zusammensetzung nach einem der Ansprüche 1 bis 5, die mindestens 10 % Gew./Vol. Magaldrat umfaßt, wenn in Suspension.

7. Zusammensetzung nach einem der Ansprüche 1 bis 5, die mindestens 18 % Gew./Vol. Magaldrat umfaßt, wenn in Suspension.

8. Zusammensetzung nach einem der Ansprüche 2 bis 7, worin der mehrwertige Alkohol 2 bis 6 freie Hydroxylgruppen enthält.

9. Zusammensetzung nach einem der Ansprüche 2 bis 7, worin der mehrwertige Alkohol Sorbit ist.

10. Verfahren zur Herstellung einer wässerigen Säureneutralisatorzusammensetzung, das das Mischen eines ausgefällten und ungetrockneten Magaldratgels, eines Aluminiumhydroxidgels mit kolloidalen Eigenschaften als erstes Fluidisierungsmittel und von Kaliumcitrat als zweites Fluidisierungsmittel umfaßt, wobei die Kombination der Fluidisierungsmittel in einer fluidisierenden Menge zur Bildung einer fluiden, resuspendierbaren pharmazeutisch annehmbaren Säureneutralisatorzusammensetzung ist.

11. Verfahren nach Anspruch 10, worin vor dem Mischen mit den Fluidisierungsmitteln das Magaldratgel in Form einer steifen und nicht-fließenden Paste ist.

12. Verfahren nach Anspruch 10, worin vor dem Mischen mit den Fluidisierungsmitteln das Magaldratgel in einer verdünnten Konzentration ist und nach dem Mischen mit den Fluidisierungsmitteln die wässerige Mischung durch Entfernen des Wassers konzentriert wird.

13. Verfahren nach Anspruch 10, das die folgenden Schritte umfaßt:
    (a) Bilden einer wässerigen Mischung, die eine fluidisierende Menge einer Kombination eines Aluminiumhydroxidgels mit kolloidalen Eigenschaften als erstes Fluidisierungsmittel und von Kaliumcitrat als zweites Fluidisierungsmittel enthält;
    (b) Konzentrieren eines ausgefällten und ungetrockneten Magaldratgels auf eine G/V Konzentration von mindestens 10 %;
    (c) zunehmendes und kontinuierliches Einbringen und Mischen des konzentrierten Magaldratgels in einen Strom der die Fluidisierungsmittel enthaltenden Mischung, wodurch das konzentrierte Gel zu einer fluiden Suspension fluidisiert wird; und
    (d) zunehmendes und kontinuierliches Einbringen und Mischen von weiterem konzentrierten Magal-

22

EP 0 178 895 B1

dratgel in einen kontinuierlich rückgeführten Strom der zunehmend konzentrierten fluidisierten Suspension, um eine fluidisierte Magaldratsuspension mit hohem Säureneutralisationsvermögen vorzusehen.

14. Verfahren zur Herstellung einer rehydratisierbaren Säureneutralisatorzusammensetzung, die bei Mischen mit Wasser eine fluide resuspendierbare pharmazeutisch annehmbare Suspension bildet, welches Verfahren das Mischen eines ausgefällten und ungetrockneten Magaldratgels, eines Aluminiumhydroxidgels mit kolloidalen Eigenschaften als erstes Fluidisierungsmittel, von Kaliumcitrat als zweites Fluidisierungsmittel und eines mehrwertigen Alkohols und das Trocknen der erhaltenen Mischung umfaßt, wobei die Kombination der Fluidisierungsmittel eine fluidisierende Menge ist.

15. Verfahren nach Anspruch 14, das die folgenden Schritte umfaßt:
(a) Bilden einer wässerigen Mischung, die einen mehrfachen Alkohol und eine fluidisierende Menge einer Kombination eines Aluminiumhydroxidgels mit kolloidalen Eigenschaften als erstes Fluidisierungsmittel und von Kaliumcitrat als zweites Fluidisierungsmittel enthält;
(b) Konzentrieren eines ausgefällten und ungetrockneten Magaldratgels auf eine G/V Konzentration von mindestens 10 %;
(c) zunehmendes und kontinuierliches Einbringen und Mischen des konzentrierten Magaldratgels in einen Strom der die Fluidisierungsmittel enthaltenden Mischung, wodurch das konzentrierte Gel zu einer fluiden Suspension fluidisiert wird; und
(d) Trocknen der Mischung des Magaldratgels und des ersten und zweiten Fluidisierungsmittels.

Patentansprüche für die Vertragsstaat AT

1. Verfahren zur Herstellung einer wässerigen Säureneutralisatorzusammensetzung, welches das Mischen eines ausgefällten und ungetrockneten Magaldratgels, eines Aluminiumhydroxidgels mit kolloidalen Eigenschaften als erstes Fluidisierungsmittel und von Kaliumcitrat als zweites Fluidisierungsmittel umfaßt, wobei die Kombination der Fluidisierungsmittel in einer fluidisierenden Menge zur Bildung einer fluiden, resuspendierbaren pharmazeutisch annehmbaren Säureneutralisatorzusammensetzung ist.

2. Verfahren nach Anspruch 1, worin vor dem Mischen mit den Fluidisierungsmitteln das Magaldratgel in Form einer steifen und nicht-fließenden Paste ist.

3. Verfahren nach Anspruch 1, worin vor dem Mischen mit den Fluidisierungsmitteln das Magaldratgel in einer verdünnten Konzentration ist und nach dem Mischen mit den Fluidisierungsmitteln die wässerige Mischung durch Entfernen des Wassers konzentriert wird.

4. Verfahren nach Anspruch 1, das die folgenden Schritte umfaßt:
(a) Bilden einer wässerigen Mischung, die eine fluidisierende Menge einer Kombination eines Aluminiumhydroxidgels mit kolloidalen Eigenschaften als erstes Fluidisierungsmittel und von Kaliumcitrat als zweites Fluidisierungsmittel enthält;
(b) Konzentrieren eines ausgefällten und ungetrockneten Magaldratgels auf eine G/V Konzentration von mindestens 10 %;
(c) zunehmendes und kontinuierliches Einbringen und Mischen des konzentrierten Magaldratgels in einen Strom der die Fluidisierungsmittel enthaltenden Mischung, wodurch das konzentrierte Gel zu einer fluiden Suspension fluidisiert wird; und
(d) zunehmendes und kontinuierliches Einbringen und Mischen von weiterem konzentrierten Magaldratgel in einen kontinuierlich rückgeführten Strom der zunehmend konzentrierten fluidisierten Suspension, um eine fluidisierte Magaldratsuspension mit hohem Säureneutralisationsvermögen vorzusehen.

5. Verfahren zur Herstellung einer rehydratisierbaren Säureneutralisatorzusammensetzung, die bei Mischen mit Wasser eine fluide resuspendierbare pharmazeutisch annehmbare Suspension bildet, welches Verfahren das Mischen eines ausgefällten und ungetrockneten Magaldratgels, eines Aluminiumhydroxidgels mit kolloidalen Eigenschaften als erstes Fluidisierungsmittel, von Kaliumcitrat als zweites Fluidisierungsmittel und eines mehrwertigen Alkohols und das Trocknen der erhaltenen Mischung umfaßt, wobei die Kombination der Fluidisierungsmittel in einer fluidisierenden Menge ist.

23

6. Verfahren nach Anspruch 5, das die folgenden Schritte umfaßt: (a) Bilden einer wässerigen Mischung, die einen mehrwertigen Alkohol und eine fluidisierende Menge einer Kombination eines Aluminiumhydroxidgels mit kolloidalen Eigenschaften als erstes Fluidisierungsmittel und von Kaliumcitrat als zweites Fluidisierungsmittel enthält;

(b) Konzentrieren eines ausgefällten und ungetrockneten Magaldratgels auf eine G/V Konzentration von mindestens 10 %;

(c) zunehmendes und kontinuierliches Einbringen und Mischen des konzentrierten Magaldratgels in einen Strom der die Fluidisierungsmittel enthaltenden Mischung, wodurch das konzentrierte Gel zu einer fluiden Suspension fluidisiert wird; und

(d) Trocknen der Mischung des Magaldratgels und des ersten und zweiten Fluidisierungsmittels.

7. Verfahren nach einem der Ansprüche 1 bis 6, worin auf Trockenbasis das Verhältnis von Magaldrat zu der Kombination der Fluidisierungsmittel 25 1 bis 2 : 1 beträgt.

8. Verfahren nach einem der Ansprüche 1 bis 6, worin auf Trockenbasis das Verhältnis des ersten Fluidisierungsmittels zum zweiten Fluidisierungsmittel 1 : 15 bis 1 : 1 beträgt.

9. Verfahren nach einem der Ansprüche 1 bis 6, worin auf Trockenbasis das Verhältnis von Magaldrat zu der Kombination der Fluidisierungsmittel etwa 8 : 1 bis etwa 4 1 und auf Trocken basis das Verhältnis des ersten Fluidisierungsmittels zum zweiten Fluidisierungsmittel 1 : 6 bis 1 : 2 beträgt.

10. Verfahren nach einem der Ansprüche 5 bis 9, worin der mehrwertige Alkohol 2 bis 6 freie Hydroxylgruppen enthält.

11. Verfahren nach einem der Ansprüche 5 bis 9, worin der mehrwertige Alkohol Sorbit ist.

12. Verfahren nach einem der Ansprüche 1 bis 11, worin das Magaldrat mindestens 10 % Gew./Vol. ausmacht, wenn in Suspension.

13. Verfahren nach einem der Ansprüche 1 bis 11, worin das Magaldrat mindestens 18 % Gew./Vol. ausmacht, wenn in Suspension.